# EUROPEAN PATENT SPECIFICATION

(11) **EP 1 802 249 B1**
(45) Date of publication and mention of the grant of the patent: **26.09.2012**
(21) Application number: 05789451.1
(22) Date of filing: 06.10.2005
(51) Int. Cl.: A61C 8/00, A61F 2/28

(54) **PROSTHETIC STRUCTURE**
PROTHESENSTRUKTUR
STRUCTURE DE PROTHÈSE

(30) Priority: 07.10.2004 SE 0402427
(43) Date of publication of application: 04.07.2007
(73) Proprietor: Dan Lundgren AB, 436 51 Hovas (SE)
(72) Inventor: LUNDGREN, Dan, S-436 51 Hovås (SE)
(74) Representative: Janson, Ronny
(86) International application number: PCT/SE2005/001481
(87) International publication number: WO 2006/038877

(56) References cited:
- EP-A- 0 393 324
- WO-A-02/062257
- WO-A1-99/22666
- DE-A1- 4 211 561
- DE-A1- 19 748 268
- DE-U1- 8 904 919
- US-A- 5 219 286
- US-A- 5 575 651
- US-A1- 2003 093 078
- US-A1- 2003 108 845
- US-A1- 2004 142 300

## Description

The invention relates to a device for the production of a prosthetic structure, according to the preamble of the appended independent claim 1.

A requirement for a prosthetic structure to correctly rest against fixtures, for instance implants, is that no significant stresses should occur in the structure when the same is connected to the fixtures. Such stresses can establish forces, which tend to displace the fixtures mutually, whereby the patient may experience them as negative. Such stresses may also lead to an incomplete alignment between co-operating support surfaces on the prosthetic structure and the respective fixture/implant, so that a tightened mounting screw can get a tendency of becoming threaded out when the structure is subjected to varying loads.

The fixtures usually comprise implants, for instance a dental implant. Usually, a model of the jaw of the patient is manufactured using implant analogues. On the same, a tubular member is releasably mounted by means of a fixing screw, which extends through the member and having a head that rests against a seating in the member. The threaded end of the fixing screw engages by a thread in the implant/the implant analogue in order to bring the support surfaces of the member and the implant analogue, for instance ring-shaped support surfaces, stably into solid contact with each other around the fixing member. Between adjacent pairs of such members along a line of implants, for instance dental implants in one of the jaws of a patient, bars are now to be bridgingly mounted on the members releasably carried by the implant analogues, without introducing any stresses. After that, the formed mounted structure is to be fixed in the substantially stressless state thereof. The intention is that the structure then may be provided with a superstructure of a conventional kind per se, for instance corresponding to a dental prosthesis and/or teeth, in order to, without problems, i.e., without introduction of stresses, subsequently be allowed to be connected to the implants in the patient by means of said members and the appurtenant fixing screws.

A previously known technique ("DynaStar") includes that the implants have cup-shaped upper surfaces and that the bars are telescopic and have ball-shaped end bodies, which are articulately received in the recesses of the implants. The ball-shaped end portions of the bars have a throughput opening each for a fixing member, which with the threaded portion thereof is received in a central threaded boring in the implant and which by means of the head thereof clamps the ball down in the recess of the implant. In the mounting operation, the telescopic bar is given a chosen length, and furthermore, the telescopic bar parts are mutually fixed by welding, for the formation of a rigid and stiff structure that may be dismounted from the implants/ /the implant analogues.

A problem of the previously known structure is, however, that the balls of the bars should rest on the cup-shaped upper ends of the implants, whereby the possibility of displacement of the connection point of the bar along the axis of the implant cannot be attained in a simple way.

Another drawback is that the structure relies on telescopic bars, which means difficulties when the distances between adjacent implants are small.

DE-A-197 48 268 describes a device for the building up of a prosthetic structure, which bridges over two implants in a patient or bridges over two implant analogues in a model of the patient. The device comprises bars, which extend between and are connected to the implants. A tubular member is releasably mounted on one end of the implant or the implant analogue in axial alignment to the same. Each tubular member carries a body, which has a throughput channel, in which the tubular member is received. The body is arranged displaceably into a chosen position along the tubular member. Cooperating pairs of surface portions of the body and the bars are substantially complementary. Means are provided for the shape-wise bonding of the ends of the bars to the respective nearby bodies. The bars have a preselected length. The ends of the bars are ball shaped and inserted into recesses in the bodies.

EP-A-0 393 324 describes a device for the building up of a prosthetic structure, which bridges over two implants in a patient or bridges over two implant analogues in a model of the patient. The device comprises bars, which extend between and are connected to the implants. A tubular member is releasably mounted on one end of the implant or the implant analogue in axial alignment to the same. Each tubular member carries a body. Cooperating pairs of surface portions of the body and the bars are substantially complementary. Means are provided for the shape-wise bonding of the ends of the bars to the respective nearby bodies. The bars are telescopic bars of adjustable length. The ends of the bars are ball shaped and inserted into recesses in the bodies.

An object of the invention is, therefore, to provide a method and a new device by means of which said drawbacks may be obviated entirely or partly.

The object is attained by the invention.

The invention is defined in the appended independent claim 1.

Embodiments of the invention are defined in the appended dependent claims.

Important features of the invention is that a body having a. throughput channel is mounted on and displaced along a tubular member, which is releasably mounted on an implant/ /implant analogue at a desired axial position along said member. The body defines the position of the connection points of the connecting bars. This position may, in other words, be set by a chosen displacement of the body along the tubular member. The tubular member and the body should co-operate by a certain friction, for instance with a threaded joint or a frictional fitting, allowing the body to be displaced along the member by means of manually pressed forces into a chosen position.

When the distances between adjacent implants/implant analogues are relatively great, it is advantageous to utilize axially extensible/shortenable ("telescopic") bars, the end portions of which are, for instance, cup-shaped, concave and co-operate with the (for instance, convex) surface portions of the bodies adapted thereto, in order to adjust the same bars to the correct length and into shape-wise bonding to the bodies. But when the distances between the implants/the implant analogues are relatively short, instead it is advantageous to utilize bars having fixed length. Thus, a bar is selected from a set or series of bars of different length. One of the two bodies to which the bar is to be joined, may have an oval shape.

In relation to the previously known structure, which also requires that the screw head and the ball have articulately co-operating surfaces having a centre of curvature that substantially coincides with the centre of curvature of the co-operating fusion faces of the ball and the implant, the invention also offers the advantage of allowing a greater range of deflection between the implant axis and the axis of the connecting bar.

In the following, the invention will be described by way of examples, reference being made to the appended drawing.
- Fig. 1: schematically shows a side view of a prosthesis as mounted on implants or implant analogues.
- Fig. 2: shows a view taken along the line II-II in Fig. 1.
- Fig. 3: schematically illustrates an implant having a tubular member mounted thereon and a body mounted thereon.
- Figs. 4 and 5: show in axial section two different embodiments of a bar.
- Fig. 6: schematically illustrates a fixed bar, as joined between two adjacent bodies, one of which is oval.
- Fig. 7: schematically illustrates an alternative embodiment of the co-operating surface sectors of the body and of the bar.
- Fig. 8: shows a view taken along the line X - X in Fig. 7.

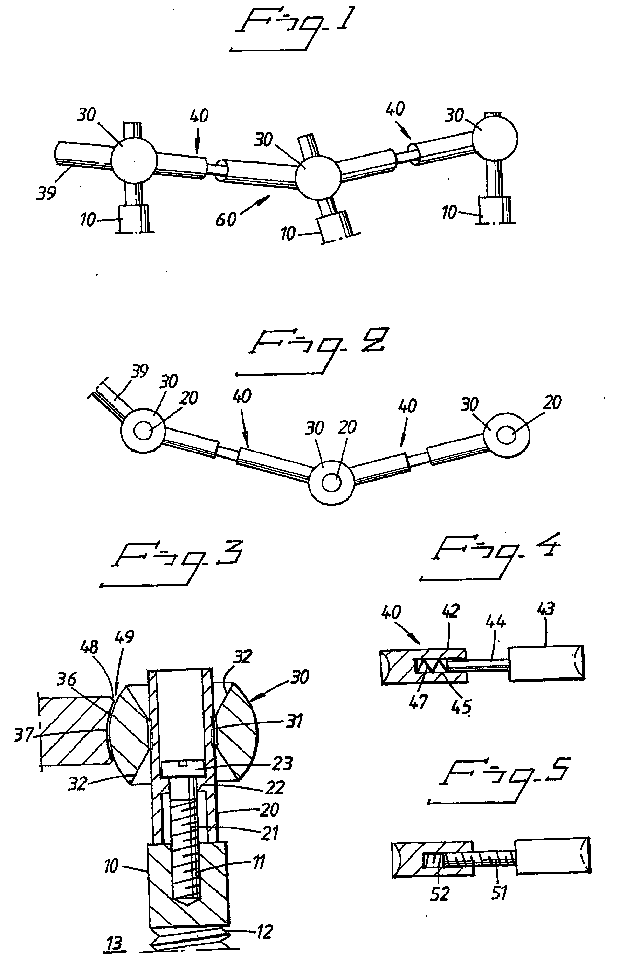

Figs. 1 and 2 illustrate a prosthetic structure carried by three implants. Each implant 10 carries a tubular member 20 that rests against the implant 10 and is releasably connected to the same by a fixing member 21, which by means of a thread engages in a threaded boring 11 in the implant 10. The member 20 has a step 22 that forms a seating for the head 23 of the fixing member 21. The tubular member 20 has a substantially constant outer cross section along the length thereof. A ball 30 has a throughput opening 31 and is threaded on the member 20. The wall of the opening 31 is, for instance, in the form of a thread, which threadably engages with a corresponding outer thread (not shown) on the outside (circumference surface) of the member 20, so that the ball 30 may be displaced axially along the member 20 and assume a substantially stable axial position along the same. As an alternative to the thread, the wall 31 of the throughput opening and/or the circumferential surface of the member 20 has scores or a surface roughness offering a limited frictional coupling between the ball and the member, whereby an operator may displace the ball 30 manually along the member 20 into a chosen position, where the ball stays. As an additional alternative, the ball and the member have a mutual fit that offers a similar frictional binding.

The throughput channel of the ball is shown to have widened sections 32 at the ends in order to facilitate for the operator to thread the ball 30 onto the member 20.

The implant 10 is shown to have a thread 12, by means of which the implant is anchored in bone tissue 13, for instance in the jawbone of a patient when the implant 10 is to support a dental prosthesis.

Figs. 1 and 2 illustrate three structures corresponding to Fig. 3, a bar 40 being shown inserted between adjacent pairs of bodies 30, which are shown in the form of balls. The bar 40 has opposite end portions 37, which are cup-shaped by a radius substantially corresponding to the radius of the balls 30. As can be seen in Figs. 1 and 2, the bars 40 are shown to be axially adjustable in respect of the length, in order to, in a relatively short state, be possible to be inserted between two adjacent balls 30, and there be expanded until the cup-shaped end surfaces thereof come into surface-extended contact with the balls on the sides thereof facing each other.

As shown in Fig. 4, the bar 40 comprises two parts 42, 43 that mutually are axially directed for linear motion, for instance by the fact that one part 43 has a protruding pin 44, which is received in a corresponding guiding channel 45 in the other part 42. Preferably, the parts 44, 45 engage by a certain limited friction against each other so that the bar 40 may be adjusted manually into chosen the length and keep this length, as inserted between a pair of balls 30. As another alternative, a spring, for instance a compression spring 47, is received in the channel 45 between the bottom thereof and the pin 44, for prestressing the bar 40 toward the maximal length thereof, preferably defined by co-operating stop faces on the two parts. In this way, an operator may compress the bar 40 before the same is inserted between two adjacent balls 30, and subsequently the bar is released and allowed to expand, under the effect of the spring 47, into contact with the respective ball.

Fig. 5 shows an axial section through another embodiment of a bar 40, comprising two parts 42, 43, one of which has a bar 44 that is received in a boring 45 in the other part 42. In Fig. 5, the bar 44 and the channel 45 are shown to have co-operating screw threads 51, 52. Alternatively, the threads 51, 52 are replaced by scores or surface irregularities allowing the parts 42, 43 to be mutually axially displaced and to keep the adjusted length up to a certain axial load.

It will be appreciated that the structure 60 shown in Fig. 1 after the building up thereof and after the fitting of the included members to each other, substantially has no stresses that tend to deform the structure, in particular the members 20 away from their set positions on the implants 10. Furthermore, it will be appreciated that the building up of the structure includes that the balls 30 are displaced along the members 20 into chosen axial positions along the members 20, i.e., to chosen distances from the implants 10. Next, the fitted structure according to Figs. 1 and 2 is fixed by the fact that the bars 40 are fixed in the adjusted length and by the fact that the bars 40 are fixed/joined to the balls 30 and the balls 30 are fixed to the members 20. The fixation is provided by means of glue joints, welded joints, soldered joints. In the case of welded joints, the parts are first jointed to each other by welding by means of welding spots and then the same are supplemented with welding seams.

In a particularly preferred embodiment, the cup-edge is chamfered in the respective end of the bar 40, such as shown at 48 in Fig. 3, in order to define, with the surface 36 of the ball 30, a circumferential V-shaped gap 49, which facilitates establishment of a welding seam or glue joint between the bar and the ball. The chamfering in the ends of the throughput channel 31 of the ball also allows facilitated accessibility to the joint between the channel wall 31 of the ball and the outer circumference of the member, for the assurance of, for instance, a welding seam.

In a few embodiments, axially adjustable bars 40 bridge over a great distance range between adjacent balls 30, but when the distance between adjacent balls 30 becomes small, it is preferred to form the bars 40 to have constant length. In order to, in a such case, be able to insert such bars 40' between two balls in a simple way, one of the balls 30 has an elliptical shape, i.e., have a varying radius around the circumference thereof in relation to the axis of the throughput channel 31, so that the ball has at least one substantially spherical bulge 38 of advanced radius R. When the bulge 36 extends substantially perpendicularly from the connecting line between two adjacent balls 30, a bar 40' of fixed length may be inserted between the balls in order to then be fitted shape-wise bondingly in between the same by turning the oval ball 30' into alignment to the axis of the bar 40'. Alternatively, the body is entirely round, but has a throughput opening that is eccentrically located in relation to the envelope surface of the body.

Fig. 1, finally, shows a ball 30 that is mounted at one end of the prosthesis, and has a cantilever arm 39, which is intended to provide an extension of the prosthesis arc that is built up past the last implant 10 in the row.

A person skilled in the art appreciates that the structure 60 does not necessarily need to be built up on the implant 10 in the jaw of a patient, but may be built up on an implant analogue in a model of the jaw of the patient. When the structure 60 has been fixed, a prosthesis is built up on the same, wherein the prosthesis, for instance, comprises tooth members and prosthesis parts, when the construction is a dental prosthesis. It will be appreciated, however, that the prosthesis shows general use, even if it primarily has been developed in order to provide dental bridge constructions and the like, which should be releasably fixed to the implant 10.

Thanks to the invention, the structure 60 is easily given a shape that does not have any built-in stresses and that, therefore, does not have any tendency to deviate from the shape it has been given in the building up on the implants/the implant analogues.

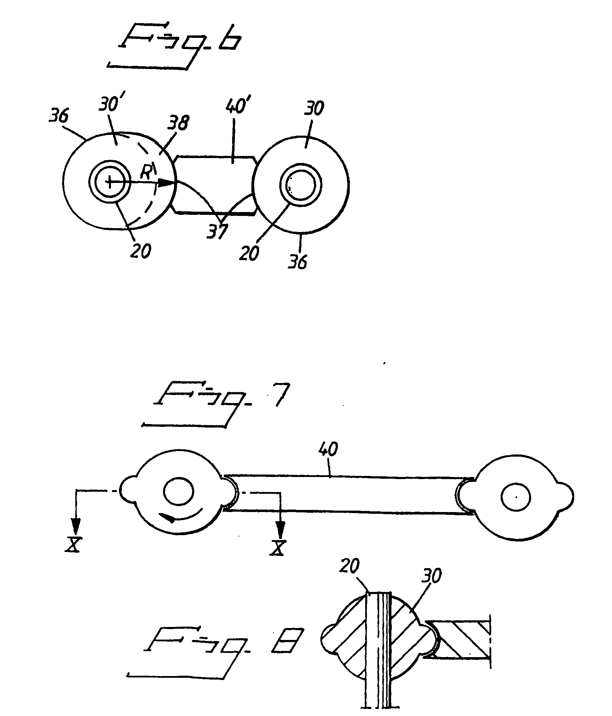

In the embodiment according to Fig. 6, where the bar has a fixed length and a concave end surface for the co-operation with a convexly rounded, preferably spherical surface sector of the body 30, and because this surface sector has a centre of curvature that coincides with the axis of rotation of the body (and the end surface of the bar has a corresponding curvature), the fitting of the bar to the body may be effected by the fact that the body is rotated while the bar is kept aligned to the axis of rotation of the body.

Suitably, the bodies have two substantially diametrically opposed surface sectors for the co-operation with a respective bar end.

In other cases (Figs. 7 and 8) , it is, in the fitting of bars having fixed length, suitable to ensure that the bar turns around the opposite end thereof that rests against an adjacent body at the same time as the freely movable end thereof follows the surface sector in question of the body during the turning motion of the body for the shape-wise bonding engagement. In this way, a shape-wise bonding between the body and the bar end is offered, also when the central portion of the bar has a fixed length corresponding to the free distance that can be adjusted between two bodies.

The concave surface portion of the bar end, for instance, is suitably cup-shaped and lacks undercut, the cup-shape receiving the convex surface portion of a radially protruding part of the body in such a way that it is possible to change the direction of the bar end in relation to the body. For instance, the concave surface has a greater radius than the convex one. When the first bar end is received on a first body, the co-operating surface portions of the second body and of the other end of the bar should be able to move into engagement with each other during turning, about in the same way as a cog and a gash of two co-operating cogwheels.

As can be seen in Fig. 3, the bar end has a concave surface 37, which co-operates with the generally spherical outer surface of a body 30 formed as a ball.

## Claims

1. Device for the building up of a prosthetic structure, which bridges over two implants (10) in a patient or bridges over two implant analogues (10) in a model of the patient, comprising a bar (40), which extends between and is connected to the implants (10), wherein
- a tubular member (20) is releasably mounted on one end of the implant/the implant analogue (10) in axial alignment to the same,
- each tubular member (20) carries a body (30), which has a throughput channel, in which the tubular member (20) is received, wherein the body (30) is arranged displaceably into a chosen position along the tubular member (20), and
- co-operating pairs of surface portions (36, 37) of the body (30) and the bar (40) are substantially complementary, wherein means (42, 45; 51, 52; 30, 36) are provided for the shape-wise bonding of the ends of the bar (40) to the respective nearby bodies (30)
**characterized in that**
- at least one bar (40) has a preselected length and its end is concavely cup-shaped, wherein at least one of the bodies (30) is rotatable around the tubular member (20) thereof and has a surface portion (38) having a greater radial distance to the axis of rotation of the body (30) around the tubular member (20) than nearby surface parts (36) of the body (30) in the direction of rotation; and
- at least one bar (40) is axially variable in length and is axially inserted between a pair of bodies (30) and is extended so that the bar ends (37) shape-wise bondingly co-operate with nearby surface portions (36) of the adjacent bodies (30).

2. Device according to claim 1, **characterized in that** the parts of the device, after mutual fitting to each other, are intended to be fixed by being fixedly connected to each other for the formation of a prosthetic structure.

3. Device according to any of the claims 1-2, **characterized in that** the body (30) is in the form of a ball.

4. Device according to claim 3, **characterized in that** the ball (30) has an elliptic shape.

5. Device according to claim 3, **characterized in that** the body (30) is round and the throughput channel of the body (30) is eccentrically located in relation to an envelope surface of the body (30).

6. Device according to claim 1, **characterized in that** a set of bars (40) of graded lengths is provided, and that the at least one bar (40) of preselected length is selected from the set to have a central axial length corresponding to the minimum distance between the pair of bodies (30) when the at least one bar (40) is aligned between the bodies (30).

## Patentansprüche

1. Vorrichtung zum Aufbau einer Prothesenkonstruktion, die zwei Implantate (10) in einem Patienten oder zwei Implantatanaloga (10) in einem Patientenmodell überbrückt und eine Stange (40) umfasst, die sich zwischen den Implantaten (10) erstreckt und mit diesen verbunden ist, worin
- ein röhrenförmiges Glied (20) lösbar an einem Ende des Implantats/Implantatanalogons (10) in axialer Ausrichtung damit befestigt ist,
- jedes röhrenförmige Glied (20) einen Körper (30) trägt, der einen Durchgangskanal aufweist, in dem das röhrenförmige Glied (20) aufgenommen ist, worin der Körper (30) in eine gewählte Position verschiebbar auf dem röhrenförmigen Glied (20) angeordnet ist; und
- zusammenwirkende Paare von Oberflächenabschnitten (36, 37) des Körpers (30) und der Stange (40) im Wesentlichen komplementär sind,
worin Mittel (42, 45; 51, 52; 30, 36) zur Formverbindung der Enden der Stange (40) mit den jeweiligen in der Nähe gelegenen Körpern (30) vorgesehen sind,
**dadurch gekennzeichnet, dass**
- zumindest eine Stange (40) eine vorab gewählte Länge und ein konkav tassenförmig geformtes Ende aufweist, worin zumindest einer der Körper (30) um sein röhrenförmiges Glied (20) drehbar ist und einen Oberflächenabschnitt (38) aufweist, der einen größeren radialen Abstand zur Drehachse des Körpers (30) um das röhrenförmige Glied (20) aufweist als die in der Nähe gelegenen Oberflächenabschnitte (36) des Körpers (30) in Drehrichtung; und
- die zumindest eine Stange (40) eine axial variable Länge aufweist und axial zwischen einem Paar von Körpern (30) eingeführt werden kann und ausgestreckt werden kann, so dass die Stangenenden (37) formverbindend mit den in der Nähe gelegenen Abschnitten (36) der benachbarten Körper (30) zusammenwirken.

2. Vorrichtung nach Anspruch 1, **dadurch gekennzeichnet, dass** die Teile der Vorrichtung nach gegenseitiger Anpassung zur Bildung einer Prothesenkonstruktion fixiert werden sollen, indem sie fest miteinander verbunden werden.

3. Vorrichtung nach einem der Ansprüche 1-2, **dadurch gekennzeichnet, dass** der Körper (30) die Form einer Kugel aufweist.

4. Vorrichtung nach Anspruch 3, **dadurch gekennzeichnet, dass** die Kugel (30) eine elliptische Gestalt aufweist.

5. Vorrichtung nach Anspruch 3, **dadurch gekennzeichnet, dass** der Körper (30) rund ist und der Durchgangskanal des Körpers (30) im Verhältnis zu einer Hüllfläche des Körpers (30) exzentrisch angeordnet ist.

6. Vorrichtung nach Anspruch 1, **dadurch gekennzeichnet, dass** ein Satz Stangen (40) abgestufter Längen vorgesehen ist und dass die zumindest eine Stange (40) vorab gewählter Länge aus dem Satz ausgewählt werden kann, so dass eine zentrale axiale Länge dem Mindestabstand zwischen dem Paar von Körpern (30) entspricht, wenn die zumindest eine Stange (40) zwischen den Körpern (30) ausgerichtet ist.

## Revendications

1. Dispositif de construction d'une structure de prothèse, qui s'étend au-dessus de deux implants (10) chez un patient, ou qui s'étend au-dessus de deux implants analogues (10) dans un modèle du patient, comprenant une barre (40) qui s'étend entre et est connectée aux implants (10), dans lequel:
un élément tubulaire (20) est monté de façon libérable sur une extrémité de l'implant/de l'analogue d'implant (10) dans un alignement axial avec celui-ci;
chaque élément tubulaire (20) porte un corps (30), qui comporte un canal de passage, dans lequel l'élément tubulaire (20) est reçu, dans lequel le corps (30) est agencé de façon déplaçable dans une position choisie le long de l'élément tubulaire (20); et
des paires coopérantes de parties de surface (36, 37) du corps (30) et de la barre (40) sont sensiblement complémentaires, dans lequel des moyens (42, 45; 51, 52; 30, 36) sont prévus pour la liaison de forme des extrémités de la barre (40) aux corps proches respectifs (30),
**caractérisé en ce que**:
au moins une barre (40) présente une longueur présélectionnée, et son extrémité est en forme de coupe concave, dans lequel au moins un des corps (30) est rotatif autour de l'élément tubulaire (20) de celle-ci et comprend une partie de surface (38) qui présente une plus grande distance radiale par rapport à l'axe de rotation du corps (30) autour de l'élément tubulaire (20) que les parties de surface proches (36) du corps (30) dans le sens de rotation; et
au moins une barre (40) présente une longueur axiale variable et est insérée axialement entre une paire de corps (30) et est étendue de telle sorte que les extrémités de barre (37) coopèrent en liaison de forme avec des parties de surface proches (36) des corps adjacents (30).

2. Dispositif selon la revendication 1, **caractérisé en ce que** les parties du dispositif, après un raccordement mutuel les unes aux autres, sont destinées à être fixées en étant connectées fixement les unes aux autres de manière à former une structure de prothèse.

3. Dispositif selon l'une quelconque des revendications 1 et 2, **caractérisé en ce que** le corps (30) se présente sous la forme d'une bille.

4. Dispositif selon la revendication 3, **caractérisé en ce que** la bille (30) est de forme elliptique.

5. Dispositif selon la revendication 3, **caractérisé en ce que** le corps (30) est rond, et la canal de passage du corps (30) est situé de façon excentrique par rapport à une surface d'enveloppe du corps (30).

6. Dispositif selon la revendication 1, **caractérisé en ce qu'**un ensemble de barres (40) de longueurs graduelles est prévu, et **en ce que** ladite au moins une barre (40) de longueur présélectionnée est sélectionnée à partir de l'ensemble de manière à présenter une longueur axiale centrale qui correspond à la distance minimum entre la paire de corps (30) lorsque ladite au moins une barre (40) est alignée entre les corps (30).
